**Europäisches Patentamt**

**European Patent Office**

**Office européen des brevets**

(11) Veröffentlichungsnummer: **0 165 505**
**A1**

## (12) EUROPÄISCHE PATENTANMELDUNG

(21) Anmeldenummer: **85106399.0**

(22) Anmeldetag: **24.05.85**

(51) Int. Cl.⁴: **A 61 B 5/02**

(30) Priorität: **24.05.84 DE 3419296**

(43) Veröffentlichungstag der Anmeldung:
**27.12.85 Patentblatt 85/52**

(84) Benannte Vertragsstaaten:
**CH DE FR GB IT LI**

(71) Anmelder: **Maier, Walter A.**
**Warmbacher Strasse 21**
**D-7888 Rheinfelden(DE)**

(72) Erfinder: **Maier, Walter A.**
**Warmbacher Strasse 21**
**D-7888 Rheinfelden(DE)**

(74) Vertreter: **WILHELMS, KILIAN & PARTNER**
**Patentanwälte**
**Eduard-Schmid-Strasse 2**
**D-8000 München 90(DE)**

(54) **Vorrichtung zur stetigen Messung des menschlichen Herzpulses.**

(57) Eine Vorrichtung zur stetigen Messung der menschlichen Pulsfrequenz hat die Form eines Armbandes (4,6) mit einem Sensor (2), einer Auswerteschaltung und einer Anzeige-Einheit. Durch einen auf den Puls reagierenden, als Membran ausgebildeten Sensor, der an der Innenseite des Armbandes unmittelbar an der Haut anliegt, wird die direkte Pulsfrequenz-Messung ermöglicht. Die Signale des Sensors werden mittels Mikroleitungen im hohlräumig ausgebildeten Armband (4) zur Feststellung und Sichtbarmachung über einen digitalen Impulsempfänger auf die Meßskala (3) der Anzeige-Einheit übertragen.

# PATENTANWÄLTE
# MENGES & PRAHL

Zugelassene Vertreter vor dem Europäischen Patentamt
Professional respresentatives before the European Patent Office
Mandataires agréés près l'Office européen des brevets

Patentanwalte Menges & Prahl, Erhardtstr 12, D-8000 München 5

Dipl.-Ing. Rolf Menges
Dipl.-Chem. Dr. Horst Prahl

Telefon (089) 2015950
Telex 529581 BIPAT d
Telegramm BIPAT München

Ihr Zeichen/Your ref.

Unser Zeichen/Our ref. M-84051 EU

Datum/Date 24.5.1985

1

---

## Vorrichtung zur stetigen Messung des menschlichen Herzpulses

---

Die Erfindung bezieht sich auf eine Vorrichtung zur stetigen Messung des menschlichen Herzpulses in Form eines Armbandes mit einem Sensor, einer Auswerteschaltung und einer Anzeige.

In den letzten Jahrzehnten hat die Sterblichkeit an Herz- und Kreislauferkrankungen allgemein ständig zugenommen. Ein hoher Prozentsatz frühzeitiger Rentenfälle sind auf Krankheiten des Kreislaufsystems zurückzuführen. Von medizinischer Seite wird daher dem Bluthochdruck der Kampf angesagt und dazu eine regelmäßige, ständige Blutdruckkontrolle gefordert.

Eine stetige Pulsmessung findet sinnvoll überall dort statt, wo menschliche Arbeit, Anstrengung und Streß bisher unkontrolliert und somit gesundheitsgefährdend bzw. ein nicht kalkulierbares Risiko sind. Durch die stetige Pulsmessung an Gliedmaßen wäre daher eine ständige Selbstkontrolle wichtiger Funktionen des pulsabhängigen Kreislaufs, von Herz- und Pulsschlag gegeben. Wünschenswert ist daher eine Vorrichtung zur stetigen und wartungsfreien Messung des menschlichen Herzpulses. Bisherige Pulsmessungen sind nach heutigem Stand der Technik entweder vom Fachmann oder nur durch umständliche und praxisfremde Anwendungsmöglichkeiten bekannt. Sie werden einer ständigen Kontrolle nicht gerecht. Die bisher in der Praxis angebotenen Meßgeräte, insbesondere solche Ausführungsformen für die Selbstmessung, werden aufgrund der Dimensionen, einer gewissen Umständlichkeit und fehlender Eleganz für die so wichtige, stetige Kontrolle vom Benutzer abgelehnt.

Eine gewisse Verbesserung demgegenüber stellt zwar schon die aus der DE-OS 29 10 302 bekannte Vorrichtung zur Messung des menschlichen Herzpulses dar, bestehend aus einem Armband und einer Sensoranordnung sowie einer Auswerteschaltung und einer dreistelligen Digitalanzeige, die sich dadurch auszeichnet, daß auf der inneren Armbandseite der menschlichen Haut zugewandt ein elastischer Behälter angebracht ist, der mit einer Flüssigkeit mit sehr kleinem Kompressionsmodul gefüllt ist, wobei die Sensoranordnung so an der der Haut abgewandten Seite angebracht wird, daß der Sensor über eine Membran die Druckschwankungen aufnimmt und die elektrischen Impulse an eine Auswertevorrichtung weitergeleitet werden, die in einem kleinen uhrähnlichen Gehäuse untergebracht ist. Als Sensor dient dabei eine Piezokeramik (Piezoxyd), dessen Fläche über eine Membran an das Medium hydraulisch gekoppelt ist, oder ein Dehnungsmeßstreifen. Die erzeugte elektrische Spannung gelangt zu einer Auswertevorrichtung, wo das Signal verstärkt, die Signalfrequenz ermittelt und dann digital durch Flüssigkristallanzeige angezeigt wird. Die Auswerteschaltung arbeitet mit einer kleinen Batterie in einem Gehäuse.

0165505

Diese Vorrichtung zeichnet sich somit durch einen relativ komplexen Aufbau, die Verwendung eines Kompressionsmediums und einer Fremdenergiequelle aus.

Aufgabe der Erfindung ist es demgegenüber, eine vereinfachte und energieunabhängige Vorrichtung zur stetigen Messung des menschlichen Herzpulses, insbesondere für die Benutzung durch Jedermann, zur Verfügung zu stellen, die alle sich aus dem ermittelten Pulsschlag ergebenden notwendigen Erkenntnisse und Maßnahmen unverzüglich ermöglicht, womit nicht nur insbesondere dem kranken Menschen eine große Unterstützung geboten wird, die medizinisch notwendig ist, sondern auch dem gesunden Menschen ein Kollaps oder Infarkt oder auch nur ein Schwächeanfall schon im Vorfeld signalisiert wird. Sie soll ihrem Träger hinsichtlich dem medizinischen, biologischen, aber auch dem psychischen Zustand entscheidende, im speziellen Fall lebenserhaltende Signale für sein Verhalten vermitteln.

Zur Lösung der gestellten Aufgabe ist somit Gegenstand der Erfindung eine Vorrichtung zur stetigen Messung des menschlichen Herzpulses, wie sie in den Ansprüchen gekennzeichnet ist.

Die erfindungsgemäße Lösung wird durch die moderne, mikroskopisch kleine Spitzentechnik möglich, deren Silicium-Chips neue technische Möglichkeiten eröffnen. Die dafür entwickelte Röntgenstrahl-Lithographie und zugehörigen Ätzverfahren ließen die Strukturen integrierter Schaltkreise noch weiter verkleinern. Die Herstellung solcher Chips und ihre Behandlung, z.B. mit Bor, ist dem Fachmann auf dem Gebiet bekannt, weshalb sich eine ausführlichere Beschreibung hier erübrigt. Dies gilt auch für ihre Vereinigung mit den für die Signalverarbeitung notwendigen elektronischen Bauteilen als geschlossene Funktionseinheiten auf Silicium-Chips. Als Beispiele für im Bereich der Mikroelektronik verwendete integrierte Schaltungen sind insbesondere die sogenannten "Gate-Arrays" zu nennen, da ihr Einsatz bereits bei relativ geringen Stückzahlen kostengünstiger ist als der Aufbau von Systemen mit Standard-IC's. Der monolithisch integrierte Drucksensor auf Siliciumbasis hat neben dem eigentlichen Sensor bereits eine Auswertelogik.

Die erfindungsgemäße Lösung lehnt sich erkennbar an herkömmliche Armbanduhren an, weshalb das Tragen der erfindungsgemäßen Meßvorrichtung keinerlei Probleme für den Benutzer ergibt.

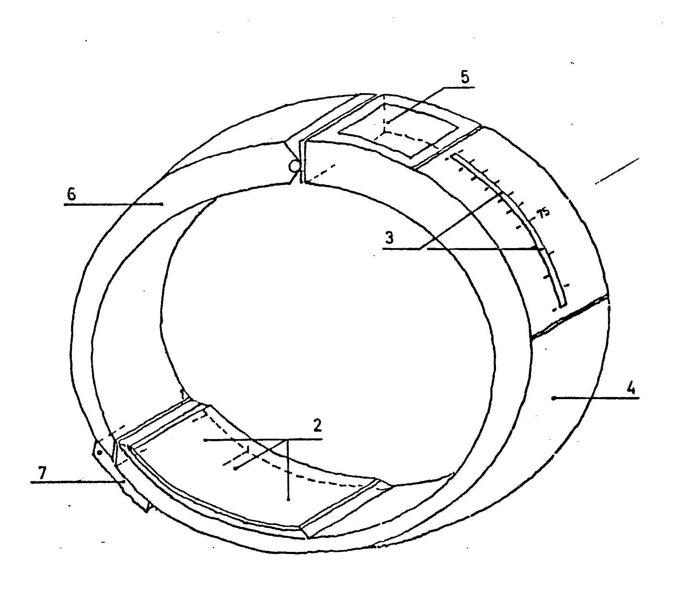

Weitere Vorteile und Merkmale ergeben sich aus der Darstellung eines bevorzugten Ausführungsbeispiels der Erfindung unter Bezugnahme auf die Figur; sie ist eine perspektivische Darstellung einer solchen Ausführungsform als Armband mit einem Ausschnitt für den Einbau einer Zeituhr.

Nach der Figur ist ein Reif 6 mit verstellbarem, beweglichem Verschluß 7 als Trägerreif, zum Beispiel aus Metall, Edelmetall oder antistatischem Kunststoff, vorgesehen. Dieser ist auf etwa 1/3 des Umfangs zu öffnen und mit dem Verschluß 7 an das jeweilige, erwünschte Körperteil, vorzugsweise den Arm, aber auch an ein Bein, stramm anzubringen. Das übrige Armband-Teil 4 von etwa 2/3 des Umfangs ist gegebenenfalls hohlräumig als Kastenprofil zur Unterbringung von Mikroleitungen, mikromechanischer und/oder elektrischer Einrichtungen, ausgebildet. Auf der Innenseite des Trägerreifs bzw. des Armband-Teils 4 ist eine auf den Pulsschlag reagierende Membran 2, also ein wie beispielhaft angegebener Sensor, eingearbeitet. Dieser gibt die Signale bzw. Frequenzen zur Anzeige weiter.

Bei der in der Figur beispielhaft dargestellten Ausführungsform weist die Anzeige des Pulsmessers eine Skala von z.B. 30 bis 200 Einheiten auf, sichtbar jeweils als

10er-Skala 3 aufgeteilt. Sie ist vorzugsweise gut ablesbar auf der Außenseite im Hohlraum des Reif-Armbandes angebracht. Ihre Abdeckung erfolgt z.B. mit Glas oder Kunststoff in antistatischem Material.

Die menschlichen Risikozonen sowohl bei hohem wie auch niedrigem Puls können durch farbiges oder Material-geändertes Sichtfeld besonders gekennzeichnet sein.

Die mit der Erfindung erzielten Vorteile bestehen insbesondere darin, daß - statt umständlicher Vorgänge, wie z.B. dem des Arztbesuches, und dem nicht selten festzustellenden, in Abhängigkeit von der menschlichen Verfassung irrationalen Meßergebnis - erst durch die erfindungsgemäße Vorrichtung eine tatsächliche, rationale, exakte und stetige Messung vom Benutzer ohne weiteres eigenes Dazutun selbst durchführbar ist.

**0165505**

Patentanwälte Menges & Prahl Erhardtstr 12. D-8000 München 5

Dipl.-Ing Rolf Menges
Dipl.-Chem.Dr Horst Prahl

Telefon (089) 2015950
Telex 529581 BIPAT d
Telegramm BIPAT München

Ihr Zeichen/Your ref

Unser Zeichen/Our ref.   M-84051 EU

Datum/Date   24.5.1985

Dipl.-Ing. Walter A. Maier

Warmbacher Straße 21

7888 Rheinfelden / Baden

---

Vorrichtung zur stetigen Messung des menschlichen Herzpulses

---

— 1 —

Patentansprüche
_____

1. Vorrichtung zur stetigen Messung des menschlichen
Herzpulses in Form eines Armbandes mit einem Sensor, einer
Auswerteschaltung und einer Anzeige, dadurch gekennzeichnet, daß als Sensor (2) eine auf den Pulsschlag reagierende Membran auf der Innenseite des Armbandes für unmittelbaren Hautkontakt freiliegt.

2. Vorrichtung nach Anspruch 1, dadurch gekennzeichnet, daß als Sensor (2) ein Feststoffmembran-Sensor auf
der Armband-Innenseite angebracht ist.

3. Vorrichtung nach Anspruch 2, dadurch gekennzeichnet, daß der Feststoffmembran-Sensor (2) ein Silicium-Chip mit den für die Signalverarbeitung notwendigen elektronischen Bauteilen als Funktionseinheit ist.

4. Vorrichtung nach einem der vorhergehenden Ansprüche, dadurch gekennzeichnet, daß das Armband-Teil (4) zur Unterbringung mikromechanischer und/oder elektrischer Einrichtungen als hohlräumiges Kastenprofil ausgebildet ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-3 216 368 (W. KOPPER) * Seite 1, Anspruch 1; Seite 2, Zeilen 10-19; Abbildung * | 1 | A 61 B 5/02 |
| X | DE-A-2 736 377 (L. CHAPUIS) * Seite 3, Zeile 14 - Seite 4, Zeile 6; Seite 6, Zeile 27 - Seite 7, Zeile 30; Seite 8, Zeilen 8-14,17-26; Abbildungen 1-3 * | 1-3 | |
| A | US-A-3 807 388 (T. ORR et al.) * Zusammenfassung; Spalte 2, Zeilen 23-34; Abbildung 2 * | 2,3 | |
| A | US-A-4 409 983 (D.E. ALBERT) * Zusammenfassung; Spalte 2, Zeilen 38-59; Spalte 3, Zeilen 25-36; Spalte 5, Zeilen 7-27; Spalte 8, Zeilen 12-22; Abbildungen 1-6 * | 1-4 | |

RECHERCHIERTE SACHGEBIETE (Int. Cl.4)

A 61 B

Der vorliegende Recherchenbericht wurde für alle Patentansprüche erstellt.

| Recherchenort | Abschlußdatum der Recherche | Prüfer |
|---|---|---|
| DEN HAAG | 13-08-1985 | RIEB K.D. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82